# EUROPEAN PATENT SPECIFICATION

(11) **EP 2 004 253 B1**
(45) Date of publication and mention of the grant of the patent: **05.01.2011**
(21) Application number: 07710486.7
(22) Date of filing: 15.02.2007
(51) Int. Cl.: A61L 31/14, A61L 31/16

(54) **STENT, INTRALUMINAL STENT DELIVERY SYSTEM, AND METHOD OF TREATING A VASCULAR CONDITION**
STENT, INTRALUMINALES STENTABGAGESYSTEM UND VERFAHREN ZUR BEHANDLUNG EINER GEFÄSSERKRANKUNG
ENDOPROTHÈSE, SYSTÈME D'APPLICATION D'ENDOPROTHÈSE INTRALUMINALE, ET PROCÉDÉ DE TRAITEMENT D'UNE PATHOLOGIE VASCULAIRE

(30) Priority: 24.03.2006 US 277419
(43) Date of publication of application: 24.12.2008
(73) Proprietor: Medtronic Vascular, Inc., Santa Rosa, CA 95403 (US)
(72) Inventor: DOTY, David, Forestville, California 95436 (US)
(74) Representative: Zimmermann & Partner
(86) International application number: PCT/US2007/062226
(87) International publication number: WO 2007/112159

(56) References cited:
- EP-A- 0 617 930
- DE-A1- 10 357 747
- US-A- 5 766 204
- US-A1- 2003 153 971

## Description

### TECHNICAL FIELD OF THE INVENTION

The present invention relates generally to stents. More particularly, the invention relates to a stent, an intraluminal stent delivery system, and a method of treating a vascular condition.

### BACKGROUND OF THE INVENTION

Balloon angioplasty has been used for the treatment of narrowed and occluded blood vessels. A frequent complication associated with the procedure is restenosis, or vessel re-narrowing. Within 3-6 months of simple angioplasty, restenosis can occur in about half of patients. To reduce the incidence of re-narrowing, several strategies have been developed. Implantable prosthetic devices, such as stents, have been used to reduce the rate of angioplasty related restenosis by about half. The use of such prosthetic devices has greatly improved the prognosis of these patients.

The objective in angioplasty is to enlarge the lumen of the affected coronary artery by radial hydraulic expansion. This is generally accomplished by inflating a balloon within the narrowed lumen of the affected artery. Radial expansion of the coronary artery may occur in several different dimensions, and is related to the nature of the plaque. Soft, fatty plaque deposits are flattened by the balloon, while hardened deposits are cracked and split to enlarge the lumen. The wall of the artery itself may also be stretched as the balloon is inflated. With simple angioplasty, the balloon may be threaded through the artery with a catheter and inflated at the place where the blood vessel is blocked. After the procedure, the balloon is then removed. The stent may then be used to support open the artery. The stent may be deployed along with the balloon or after the balloon is removed.

The stent may be formed from a generally tubular body that can be expanded from a collapsed state into a deployed state. The stent body may include a plurality of elongated element lengths (e.g., wire lengths, or the like) that are connected together to permit the stent body to be expanded. The stent may be coupled to a deployment system (e.g., a catheter) in a collapsed state. For example, the stent may be compressed within a lumen formed within a catheter or onto a catheter balloon. The catheter including the stent may be then advanced endovascularly (or within another vessel type) to the afflicted region of the body passage. While fed through the vessel, the stent remains in the collapsed state.

Once the stent has reached the afflicted region in the body passage, it may be expanded radially outward into the deployed state. The stent may be expanded into its deployed state by inflating the catheter balloon so that expansion of the stent is achieved simultaneously with the inflation of the balloon. Alternatively, the stent may be manufactured from a resilient material such that when it is collapsed, the stent may naturally expand from a "tense" collapsed state into a "relaxed" deployed state. In such a case, the stent self-expands as it is removed from the catheter lumen. Regardless of the type of stent, the radial strength of the stent should be sufficient to withstand restenosis in order to maintain vascular patency. Certain stents (e.g., non-metallic, bioabsorbable types) lack sufficient radial strength under stressful conditions (e.g., high blood pressure, bodily movements, etc.). As such, it would be desirable to provide a bioabsorbable stent with an improved radial strength.

Given that the stent deployment system typically includes a number of parts, a reduced collapsed stent profile size contributes to a reduced size in the deployment system. As such, numerous benefits may be provided by a reduction in stent and (potentially) deployment system size. For example, as the stent is advanced to the site of deployment, it may encounter a sometimes tortuous and narrow network of vessels. Smaller sized stents and deployment systems may facilitate easier negotiation of such vessel networks. Other benefits of reducing the size of the deployment system may include less disruption of an atheroma and plaque that could lead to emboli, less disruption of blood flow, less likelihood of vessel wall damage, and reduced vessel puncture size for intraluminal access. Accordingly, it would be desirable to minimize the stent collapsed profile size. DE 103 57 747 describes a biodegradable stent including a shape-memory polymer material .EP 0 617 930 describes an expandable endovascular stent having a double-walled sleeve filled with a photocurable material.

Accordingly, it would be desirable to provide a stent, an intraluminal stent delivery system, and method of treating a vascular condition that would overcome the aforementioned and other disadvantages.

### SUMMARY OF THE INVENTION

In view of the above astent according to claim 1 and a stent delivery system according to claim 6 are provided.

The foregoing and other features and advantages of the invention will become further apparent from the following detailed description of the presently preferred embodiments, read in conjunction with the accompanying drawings.

### BRIEF DESCRIPTION OF THE DRAWINGS

**FIG. 1** illustrates an intraluminal stent delivery system in accordance with the present invention;

**FIG. 2** illustrates a stent in accordance with the present invention;

**FIG. 2A** illustrates a cross section of a portion of the stent illustrated in **FIG. 2****;**

**FIG. 3** illustrates a cross-section of the stent of **FIG. 2** shown deployed in a vessel, in accordance with the present invention; and

**FIG. 4** illustrates a flowchart of a method of treating a vascular condition, in accordance with one embodiment of the present invention.

### DESCRIPTION OF THE PRESENTLY PREFERRED EMBODIMENTS

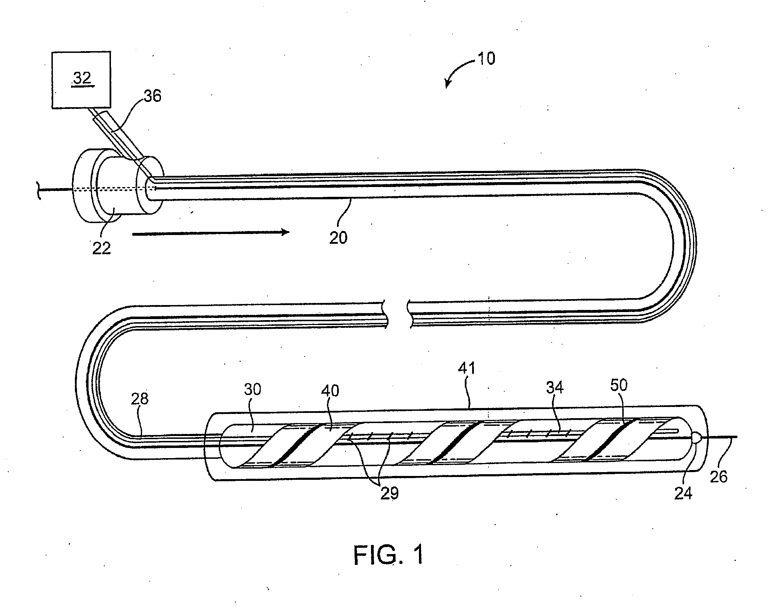

Referring to the drawings, which are not necessarily drawn to scale and wherein like reference numerals refer to like elements, **FIG. 1** is a perspective view of an intraluminal stent delivery system in accordance with one embodiment of the present invention and shown generally by numeral **10.** System **10** includes a catheter **20,** a balloon **30** operably attached to the catheter **20,** and a stent **40** disposed on the balloon **30.** Stent **40** (shown in a compressed configuration) remains compressed on the balloon **30** during advancement through the vasculature. The compressed stent **40** includes a small profile (i.e., cross-sectional size). In one embodiment, a sheath **41** may be disposed on the stent **40** to protect the stent **40** as well as the vessel walls during advancement.

Although the devices described herein are primarily done so in the context of deployment within a blood vessel, it should be appreciated that intravascular and/or implantable prosthetic devices in accordance with the present invention may be deployed in other vessels, such as a bile duct, intestinal tract, esophagus, and airway.

The term "biodegradable" refers to substances that degrade (e.g., via hydrolysis) to at least a certain extent within the body. Biodegradable substances are biocompatible and preferably incur a reduced inflammatory response. A "radial" direction is one that is perpendicular to the axis of a vessel.

In one embodiment, catheter **20** includes an elongated tubular member manufactured from one or more polymeric materials. In another embodiment, catheter **20** includes a metallic reinforcement element. In some applications (such as smaller, more tortuous vessels), the catheter is constructed from very flexible materials to facilitate advancement into intricate access locations. Numerous over-the-wire, rapid-exchange, and other catheter designs are known and may be adapted for use with the present invention. Catheter **20** can be secured at its proximal end to a suitable Luer fitting **22,** and may include a distal rounded end **24** to reduce harmful contact with a vessel. Catheter **20** can be manufactured from a material such as a thermoplastic elastomer, urethane, polymer, polypropylene, plastic, ethelene chlorotrifluoroethylene (ECTFE), polytetrafluoroethylene (PTFE), fluorinated ethylene propylene copolymer (FEP), nylon, Pebax® resin, Vestamid® nylon, Tecoflex® resin, Halar® resin, Hyflon® resin, Pellathane® resin, combinations thereof, and the like. Catheter **20** includes an aperture formed at the distal rounded end **24** allowing advancement over a guidewire **26.**

Balloon **30** may be any variety of balloons or other devices capable of expanding the stent **40** (e.g., by providing outward radial forces). Balloon **30** may be manufactured from any sufficiently elastic material such as polyethylene, polyethylene terephthalate (PET), nylon, or the like. Those skilled in the art will recognize that the stent **40** may be expanded using a variety of means and that the present invention is not limited to balloon expansion.

In one embodiment, an optical fiber **28** is positioned adjacent the balloon **30** and the distal rounded end **24.** The main body of the optical **fiber 28** is substantially straight and extends through an inflation lumen of the balloon **30** and along the length of catheter **20,** terminating proximally at a connecter arm at the Luer fitting **22.** Optical fiber **28** is operably connected to a light source **32** via its proximal portion **36.** In one embodiment, the distal portion **34** of the optical fiber **28** adjacent the stent **40** is abraded, as shown by hash marks **29,** to diffuse light from the optical fiber **28** in multiple directions.

In another embodiment, the optical fiber **28** is attached outside the catheter or, alternatively, freestanding or as part of another medical device. In another embodiment, the optical fiber **28** may be any material or apparatus capable of emitting light and may be connected to a light source by any means of conveying light to the stent. For example, a light source running under its own power source (e.g., a battery) can be positioned adjacent the balloon **30** and distal rounded end **24.**

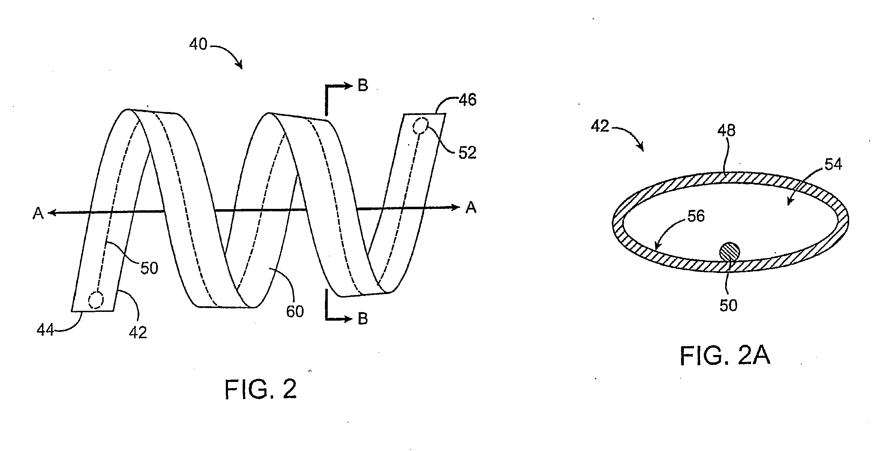

Referring to **FIG. 2****,** an assembled stent **40** in a deployed configuration is shown. In one embodiment, the stent **40** is a generally tubular structure including a passageway that extends along a longitudinal axis **A.** Stent 40 can be configured for various lengths when in the deployed configuration. In one embodiment, the length of stent 40 is predetermined based on the dimensions of the treatment site.

Stent **40** includes a flexible biodegradable elongate member **42,** which is shaped in a coiled configuration. Referring to FIG. 2A, elongate member **42** comprises an elongate member wall 48 forming a cavity 54. In one embodiment, elongate member 42 has a generally oval cross section. In another embodiment, elongate member 42 may have a generally circular cross section. Elongate member 42 can be manufactured from numerous biodegradable materials such as a thermoplastic material of poly-lactic acid (PLA), polyglycolyic acid (PGA), and/or collagen, which demonstrate high biocompatibility with reduced inflammatory response. Those skilled in the art will recognize that the size, geometry, and constituent material of the elongate member **42** may vary from the description and illustrations provided herein.

A biodegradable reinforcing member **50** is positioned adjacent the elongate member **42.** Reinforcing member **50** provides an outward radial force for supporting the stent **40** in the deployed configuration. In one embodiment, reinforcing member **50** is a biodegradable magnesium wire positioned along an inner surface **56** of the elongate member **42.** Alternatively, the reinforcing member **50** can be positioned outside or integrated into the elongate member wall **48.** In one embodiment, the reinforcing member **50** is manufactured from a resilient material for providing radial force (i.e., to resist restenosis). Reinforcing member **50** is positioned along the length of the elongate member **42** in the coiled configuration. The end portions of the reinforcing member **50** can be shaped to reduce sharp edges. In one embodiment, the end portions form hoops or rings **52.** Those skilled in the art will recognize that the size, geometry, number, and constituent material of the reinforcing member **50** may vary from the description and illustrations provided herein. For example, in another embodiment, the reinforcing member may be struts, wires, mesh, and the like, for supporting the stent **40.**

Cavity **54** of the elongate member **42** is at least partially filled with a biodegradable, photo-curable polymer **60.** The end portions **44, 46** of the elongate member **42** are sealed to retain the photo-curable polymer **60.** The end portions 44, 46 may be sealed by any means known in the art such as, for example, thermal sealed, bonded, clipped, and the like, to retain the photo-curable polymer **60.** While the stent is in the compressed configuration, mounted on the balloon **30,** the polymer **60** is in a fluid pre-polymer form, such as a liquid, gel, and the like. In one embodiment, the pre-polymer comprises a cross-linked hydrophilic polymer, commonly known as hydrogels. In one embodiment, the polymer **60** also includes a photoinitiator, such as eosin Y, which accelerates polymerization of the pre-polymer to the polymer **60.** Those skilled in the art will recognize that numerous compounds may be added to the polymer **60** to alter its curative properties.

The pre-polymer liquid contained within cavity **54** is flexible. As such, in one embodiment, mounting of the stent **40** on the balloon **30** is performed with a mechanical wrapping device or other similar device for wrapping the stent in a helical fashion. Mounting the stent **40** tightly around the balloon **30** provides a reduced collapsed profile size.

After the stent **40** is deployed and exposed to light, the liquid pre-polymer polymerizes or "cures" *in vivo* into the polymer **60.** The polymerization of the liquid pre-polymer solidifies the pre-polymer to form the cured polymer **60.** The cured polymer **60,** along with the reinforcing member **50,** supports the elongate member **42** at the treatment site. In one embodiment, the cured polymer **60** and the reinforcing member **50** supports the elongate member **42** in the radial direction to counteract vasoconstriction. The degree of the radial strength and support provided by the cured polymer depends on the nature of the cured polymer **60.** For example, an epoxy or acrylic polymeric material may provide greater radial strength than a hydrogel.

The stent **40** includes at least one therapeutic agent. The therapeutic agent is integrated with the polymer **60** thereby allowing elution as the elongate member **42** degrades. It should be noted that any polymer(s) incorporated in a therapeutic agent may or may not be the same as the biodegradable, photo-curable polymer **60.**

The therapeutic agent comprises one or more drugs, polymers, a component thereof, a combination thereof, and the like. For example, the therapeutic agent can include a mixture of a drug and a polymer as known in the art. Some exemplary drug classes that may be included are antiangiogenesis agents, antiendothelin agents, antimitogenic factors, antioxidants, antiplatelet agents, antiproliferative agents, antisense oligonucleotides, antithrombogenic agents, calcium channel blockers, clot dissolving enzymes, growth factors, growth factor inhibitors, nitrates, nitric oxide releasing agents, vasodilators, virus-mediated gene transfer agents, agents having a desirable therapeutic application, and the like. Specific examples of drugs include abciximab, angiopeptin, colchicine, eptifibatide, heparin, hirudin, lovastatin, methotrexate, rapamycin, streptokinase, taxol, ticlopidine, tissue plasminogen activator, trapidil, urokinase, zotarolimus, and growth factors VEGF, TGF-beta, IGF, PDGF, and FGF.

In one embodiment, the therapeutic agent polymer provides a matrix for incorporating the drug within a coating, or may provide means for slowing the elution of an underlying therapeutic agent when it comprises a cap coat or is incorporated into the photo-curable polymer. It should be noted that the polymer(s) of the therapeutic agent is not necessarily the same compound as the photo-curable polymer **60.** Some exemplary biodegradable polymers that may be adapted for use with the present invention include, but are not limited to, polycaprolactone, polylactide, polyglycolide, polyorthoesters, polyanhydrides, poly(amides), poly(alkyl-2-cyanocrylates), poly(dihydropyrans), poly(acetals), poly(phosphazenes), poly(dioxinones), trimethylene carbonate, polyhydroxybutyrate, polyhydroxyvalerate, their copolymers, blends, and copolymers blends, combinations thereof, and the like.

Solvents are used to dissolve the therapeutic agent and polymer to comprise a therapeutic agent coating solution. Some exemplary solvents that may be adapted for use with the present invention include, but are not limited to, acetone, ethyl acetate, tetrahydrofuran (THF), chloroform, N-methylpyrrolidone (NMP), methylene chloride, and the like.

Those skilled in the art will recognize that the nature of the drug and polymer may vary greatly and are typically formulated to achieve a given therapeutic effect, such as limiting restenosis, thrombus formation, hyperplasia, etc. Once formulated, a therapeutic agent solution (mixture) comprising the coating may be applied to the stent **40** by any of numerous strategies known in the art including, but not limited to, spraying, dipping, rolling, nozzle injection, and the like. Numerous strategies of applying the coating in accordance with the present invention are known in the art. In another embodiment, the therapeutic agent may be incorporated within the photocurable polymer **60.**

In one embodiment, two or more therapeutic agents are incorporated into the stent and are released having a multiple elution profile. For example, a first therapeutic agent disposed on the elongate member **42** is released to reduce inflammation. The first agent may be released on a short-term basis to overcome surgical trauma of the treatment. In this embodiment, the second therapeutic agent is disposed in the photo-curable polymer **60** for reducing endovascular restenosis. As the elongate member **42** biodegrades, the second therapeutic agent is released on a longer-term basis.

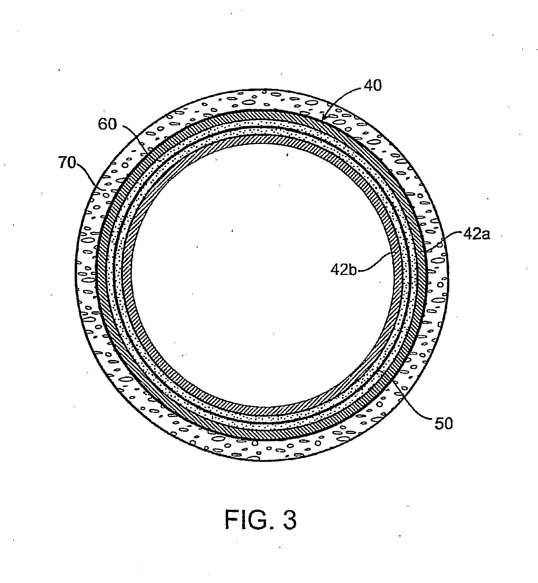

**FIG. 3** illustrates the stent **40** in cross-section deployed within a vessel **70,** taken along line **B-B** of **FIG. 2****.** An outer portion of the elongate member **42a** contacts an inner wall of the vessel **70.** Outer portion **42a** of the elongate member **42** is supported by the reinforcing member **50,** polymer **60,** and inner portion **42b** of the elongate member **42,** thereby providing axial strength to the stent **40.**

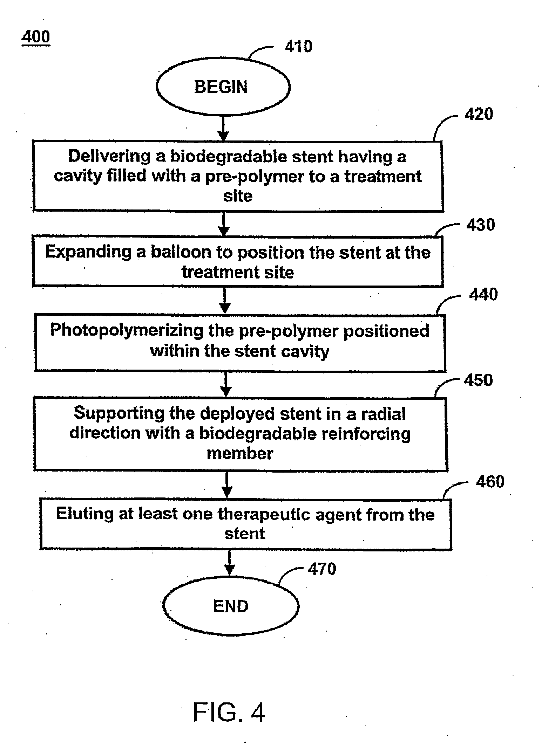

**FIG. 4** illustrates a flowchart of a method **400** of treating a vascular condition, in accordance with one embodiment of the present invention. The present description relates to the treatment of a vascular condition, which in this case is an ischemic blood vessel including a vulnerable plaque. The method begins at step **410.**

At step **420,** the stent **40** is delivered to a treatment site with the catheter **20.** In one embodiment, catheter **20** is advanced to treatment site over a pre-positioned guidewire **26.** In one embodiment, at least one radiopaque marker may be disposed on the stent **40,** catheter **20,** and or component thereof to allow *in situ* visualization and proper advancement, positioning, and deployment of the stent **40.** The market(s) may be manufactured from a number of materials used for visualization in the art including radiopaque materials such as, for example, platinum, gold, tungsten, metal, metal alloy, and the like. Marker(s) may be visualized by fluoroscopy, IVUS, and other methods known in the art. Those skilled in the art will recognize that numerous devices and methodologies may be utilized for positioning an intraluminal stent in accordance with the present invention.

At step **430,** once the stent **40** is properly positioned at the treatment site, the balloon **30** and stent **60** are expanded radially into contact with the vessel wall. Balloon **30** is expanded by addition of fluid within its lumen. In one embodiment, a sheath **41,** covering stent **40** during delivery to the treatment site, is retracted prior to expanding balloon **30.** At step **440,** the pre-polymer positioned within the stent is polymerized. In one embodiment, light is passed distally from the light source **32** via the optical fiber **28** toward the deployed stent **40.** As previously described, the light is diffused adjacent the stent **40** to provide light exposure to the pre-polymer. Light is administered until the pre-polymer has nearly or fully polymerized or cured. The intensity, wavelength, and duration of light exposure can depend on factors such as, for example, the size of the elongate member **42** and amount of pre-polymer. Those skilled in the art will recognize that the strategy for diffusing and delivering the light energy to the pre-polymer may vary from the description and illustrations provided herein. For example, different pre-polymers and stent geometries may require alternative light sources from those described herein.

At step **450,** the deployed stent is supported in a radial direction. In one embodiment, the polymerized polymer **60** serves to set the stent **40** in the deployed position and provide radial support. In another or the same embodiment, the biodegradable reinforcing member 50 provides additional support to the deployed stent **40.** Once the photopolymerization is completed, balloon **30** is deflated and removed along with the catheter **20** and guidewire **26** leaving the deployed stent **40** at the treatment site.

At step **460,** at least one therapeutic agent is eluted from the stent. In one embodiment, a single therapeutic agent is in the stent **40,** polymer **60.** In another embodiment, as previously described, two or more therapeutic agents are released with a multiple elution profile. Method **400** ends at step **470.**

## Claims

1. A biodegradable stent (40) comprising:
a biodegradable flexible elongate member (42) including an elongate member wall (48) surrounding a cavity (54), wherein the biodegradable elongate member (42) comprises a coiled configuration and at least one end portion (44, 46) of the elongate member (42) is sealed;
a biodegradable reinforcing member (50) positioned within or adjacent the elongate member wall (48) to support the biodegradable elongate member (42); and
a biodegradable photo-curable polymer (60) positioned within the cavity (54) of the elongate member (42),
the stent (40) further comprising at least one therapeutic agent integrated with the polymer (60).

2. The stent of claim 1 wherein the biodegradable photo-curable polymer (60) provides support to the stent (40) in a radial direction when in a deployed configuration.

3. The stent of claim 1 wherein the at least one therapeutic agent is eluted with a predetermined profile.

4. The stent of claim 1 wherein the reinforcing member (50) comprises a magnesium wire.

5. The stent of claim 1 further comprising a photoinitiator positioned within the cavity (54).

6. An intraluminal stent delivery system (10) comprising:
a catheter (20);
an inflatable member (30) operably attached to the catheter (20); and
a biodegradable stent (40) according to any of the preceding claims.

7. The system of claim 6 wherein the reinforcing member (50) comprises a coiled member.

8. The system of claim 6 wherein the biodegradable photo-curable polymer (60) provides axial support to the stent (40).

9. The system of claim 6 further comprising at least one fiber optic member (28) positioned adjacent the elongate member (42), wherein the fiber optic member (28) is operably attached to a light source (32) for polymerizing the photocurable polymer (60).

10. The system of claim 9 wherein the at least one fiber optic member (28) comprises a light diffusing member (34) for radially diffusing light.

11. The system of claim 6 further comprising a photoinitiator positioned within the elongate member (42).

## Patentansprüche

1. Biologisch abbaubarer Stent (40), umfassend:
ein biologisch abbaubares, flexibles, längliches Element (42), das eine längliche Elementwand (48) enthält, die einen Hohlraum (54) umgibt, wobei das biologisch abbaubare, längliche Element (42) eine spiralförmige Konfiguration umfasst und mindestens ein Endabschnitt (44, 46) des länglichen Elements (42) versiegelt ist;
ein biologisch abbaubares Verstärkungselement (50), das innerhalb oder neben der länglichen Elementwand (48) positioniert ist, um das biologisch abbaubare längliche Element (42) zu stützen; und
ein biologisch abbaubares, lichthärtbares Polymer (60), das innerhalb des Hohlraumes (54) des länglichen Elements (42) positioniert ist;
wobei der Stent (40)des Weiteren mindestens ein therapeutisches Mittel umfasst, das mit dem Polymer (60) integriert ist.

2. Stent nach Anspruch 1, wobei das biologisch abbaubare, lichthärtbare Polymer (60) eine Stütze für den Stent (40) in radialer Richtung bereitstellt, wenn er sich in der entfalteten Konfiguration befindet.

3. Stent nach Anspruch 1, wobei das mindestens eine therapeutische Mittel mit einem im Voraus bestimmten Profil eluiert wird.

4. Stent nach Anspruch 1, wobei das Verstärkungselement (50) einen Magnesiumdraht umfasst.

5. Stent nach Anspruch 1, des Weiteren umfassend einen Photoinitiator, der innerhalb des Hohlraumes (54) positioniert ist.

6. Abgabesystem für einen intraluminalen Stent (10), umfassend:
einen Katheter (20);
ein aufblähbares Element (30), das betriebsbereit an dem Katheter (20) befestigt ist; und
einen biologisch abbaubaren Stent (40) nach einem der vorangehenden Ansprüche.

7. System nach Anspruch 6, wobei das Verstärkungselement (50) ein spiralförmiges Element umfasst.

8. System nach Anspruch 6, wobei das biologisch abbaubare, lichthärtbare Polymer (60) eine axiale Stütze für den Stent (40) bereitstellt.

9. System nach Anspruch 6, des Weiteren umfassend mindestens ein faseroptisches Element (28), das neben dem länglichen Element (42) positioniert ist, wobei das faseroptische Element (28) betriebsbereit an einer Lichtquelle (32) befestigt ist, um zum Polymerisieren des lichthärtbaren Polymers (60) zu dienen.

10. System nach Anspruch 9, wobei das mindestens eine faseroptische Element (28) ein Lichtstreuungselement (34) zum radialen Streuen von Licht umfasst.

11. System nach Anspruch 6, des Weiteren umfassend einen Photoinitiator, der innerhalb des länglichen Elements (42) positioniert ist.

## Revendications

1. Stent biodégradable (40), qui comprend :
un élément allongé, flexible et biodégradable (42) comprenant une paroi d'élément allongé (48) entourant une cavité (54), l'élément allongé biodégradable (42) comprenant une configuration enroulée et au moins une partie d'extrémité (44, 46) de l'élément allongé (42) est fermée ;
un élément de renforcement biodégradable (50) positionné à l'intérieur de ou adjacent à la paroi d'élément allongé (48) pour supporter l'élément allongé biodégradable (42) ; et
un polymère photodurcissable et biodégradable (60) positionné à l'intérieur de la cavité (54) de l'élément allongé (42),
le stent (40) comprenant en outre au moins un agent thérapeutique intégré dans le polymère (60).

2. Stent selon la revendication 1, le polymère photodurcissable et biodégradable (60) fournissant un support au stent (40) dans une direction radiale lorsqu'il se trouve dans une configuration déployée.

3. Stent selon la revendication 1, au moins un agent thérapeutique étant élué avec un profil prédéterminé.

4. Stent selon la revendication 1, l'élément de renforcement (50) comprenant un fil de magnésium.

5. Stent selon la revendication 1, qui comprend en outre un photoinitiateur positionné à l'intérieur de la cavité (54).

6. Système de mise en place intraluminale de stent (10) qui comprend :
un cathéter (20) ;
un élément gonflable (30) fixé de manière opérationnelle au cathéter (20) ; et
un stent biodégradable (40) selon l'une quelconque des revendications précédentes.

7. Système selon la revendication 6, l'élément de renforcement (50) comprenant un élément enroulé.

8. Système selon la revendication 6, le polymère photodurcissable et biodégradable (60) fournissant un support axial pour le stent (40).

9. Système selon la revendication 6, qui comprend en outre au moins un élément de fibre optique (28) positionné adjacent à l'élément allongé (42), l'élément de fibre optique (28) étant fixé de manière opérationnelle à une source de lumière (32) pour polymériser le polymère photodurcissable (60).

10. Système selon la revendication 9, le ou les éléments de fibre optique (28) comprenant un élément diffusant la lumière (34) destiné à diffuser la lumière radialement.

11. Système selon la revendication 6, qui comprend en outre un photoinitiateur positionné à l'intérieur de l'élément allongé (42).
